# EUROPEAN PATENT APPLICATION

(11) **EP 3 340 233 A1**
(43) Date of publication of application: **27.06.2018**
(21) Application number: 17380022.8
(22) Date of filing: 31.10.2017
(51) Int. Cl.: G10G 5/00

(54) **DEVICE FOR THE LEGS OF CELLO, VIOLA DA GAMBA AND DOUBLE BASS PERFORMERS**

(30) Priority: 03.11.2016 ES 201600745
(71) Applicant: López Fernández, Jose Luis, 28009 Madrid (ES)
(72) Inventor: López Fernández, Jose Luis, 28009 Madrid (ES)

(57) **Abstract**

It is a cushion device to be placed in the Cello, Viola da gamba and Double Bass performers' legs. The object of the invention is to optimize the acoustic qualities of the instruments improving qualitatively and quantitatively their sound. It prevents future injuries. It prevents beginners to acquire bad postures and shows them the correct support point of the instrument.

This device is characterized by the following pieces: a cushion body (1), and a leg fastening system with a closing and opening system. The cushion body (1) can be made of a plastic substance with acoustic properties that buffer the transmission of the instruments' vibration to the musicians' body. The device is attached to the lower part of the legs and its upper part allows the instrument to rest naturally on the corresponding points of support (12, 13, 14, 15, 16 y 17). The device will be manufactured in different sizes attending to the morphological characteristics of the instrument, and the age and physical constitution of the musician.

## Description

### Field of technology

The present invention consists of a device designed for Cello, Viola da gamba and Double Bass performers which optimizes the acoustic qualities of these instruments, qualitatively improving their sonority. At the same time, it provides comfort during musical training and prevents future injuries as well as the fatigue produced after hours of study. Further, it prevents the acquisition of bad postures due to the pain in the legs produced by the instrument during the first years of learning, usually during childhood. Finally, it shows students the correct support point of the instrument.

### The state of the art

So far a device has been designed that is meant to prevent the occupational illnesses that are linked to the learning of a musical instrument such as the cello - my utility model ES201600745U -. But there is no knowledge in the previous state of the technology of any other device to be placed on the legs of Cello, Viola da gamba and Double Bass performers which can solve at the same time the following problems resulting from the learning of a string instrument which is laid on or between the legs: a) inhibiting the loss of vibration due to the direct rest of the Cello, Viola da gamba or Double bass on or between the Cellist, Viola da gamba or Double bass players' legs, with a clear qualitative improvement of the instrument sonority; b) providing comfort to the musical practice; c) preventing future injuries; d) avoiding the pain due to the continuous holding of the instrument between the legs for long periods of practice, inherent to the study of an instrument; e) avoiding during the initial period of learning the instrument, which usually takes place in childhood, that the student acquires a bad posture due to the pain caused by the resting of the instruments on the legs of beginners; f) showing the student the correct support point of the instrument.

### Explanation of the invention

The present invention, as expressed in the statement of this descriptive memory, refers to a device for the Cello, Viola da Gamba and Double Bass performers' legs, which has been designed and produced in order to give an answer to the above mentioned problems.

Thereby the device attends to the prevention of the occupational illnesses linked to the musical practice with the Cello, a problem to which my utility model ES201600745U gave an answer and whose priority is demanded for claims 2, 3 y 4 below. It also attends to the possible pain in the legs due to the continuous holding of the instrument during the musical practice with the Viola da Gamba or the Double Bass.
In this first claim, the device is used as a protection. It is designed to be used with all musical instruments which are laid on or between the legs, as the Cello, Viola da Gamba and Double Bass, providing performers with comfort in the musical practice, preventing future injuries and avoiding the pain due to the long hours of study.

It consists of a circular cushion structure with a semi-elliptical surface which can be attached to the thigh where the cello leans on and allows its natural resting. This structure contains a semi-rigid disc adjustable, which protects the muscular system from the pain caused by the pressure exerted by the cello's corner.

It avoids that in the initial period of learning the instrument, which usually occurs in childhood, the student acquires a bad posture due to the pain caused by the instrument resting on the legs of beginners.

It shows the student the correct support point of the instrument on the legs.

The device is planned to be manufactured in different sizes, according to the morphological characteristics of the instrument, the age and the physical constitution of the Cello, Viola da gamba or Double Bass player.

In a different embodiment, the device can be produced as an acoustic cushion that inhibits the vibration loss that occurs when the Cello, Viola da gamba or Double bass rests directly on or between the legs of the cellist, Viola da gamba player or Double Bass player, optimizing the acoustic qualities of the instrument, achieving a clear qualitative improvement of the instrument sonority.

This version consists of a cushion body with an inner belt that can be attached to the leg and which, thanks to its upper part, allows the instrument to rest naturally. This body contains a plastic substance whose acoustic properties buffer the vibration transmission of the instrument to the musician's body, improving the instrument sonority.

This structure can perfectly adapt to the Cello, Viola da gama and Double Bass players' legs thanks to a fabric band that can be adapted to the leg's size and which is secured through a fast and easy closing and opening system.

For the embodiment where it is only employed as a protector, the material used for the central piece has to be soft but firm. This material should be ideally a gel.

For the embodiment where it is not only employed as a protector but also as an acoustic cushion, the material used for the central piece will be a plastic material with acoustic proprieties. In an ideal realization this material is polyurethane foam.

### Brief description of the drawings

Figure 1 shows a protector device for the cellists' thig according to the utility model n° ES201600745U with a perspective view from above the device object of the invention.
Figure 2 shows a protector device for the cellists' thig according to the utility model n° ES201600745U with a lateral perspective view of the device object of the invention.
Figure 3 shows an acoustic cushion for the Cellist, Viola da gamba player or Double bass players' legs with a perspective view from above the device object of the invention.
Figure 4 shows an acoustic cushion for the Cellist, Viola da gamba player or Double Bass players'legs with a lateral perspective view from above the device object of the invention.

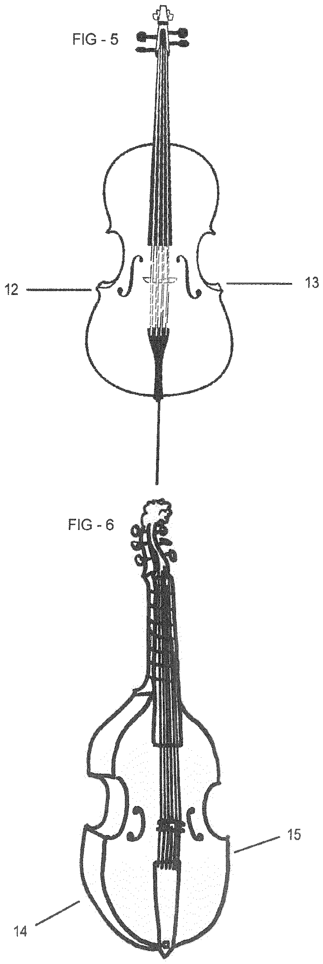

Figure 5 shows a frontal perspective view from the Cello specifying which are the support points on the legs for the instrument.
Figure 6 shows an oblique perspective view from the Viola da gamba specifying which are the support points on the legs for the instrument.

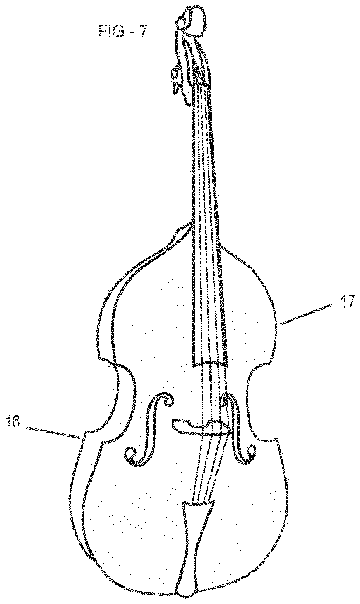

Figure 7 shows an oblique perspective view from the Double Bass specifying which are the support points on the legs for the instrument.

### Detailed presentation of an execution mode of the invention

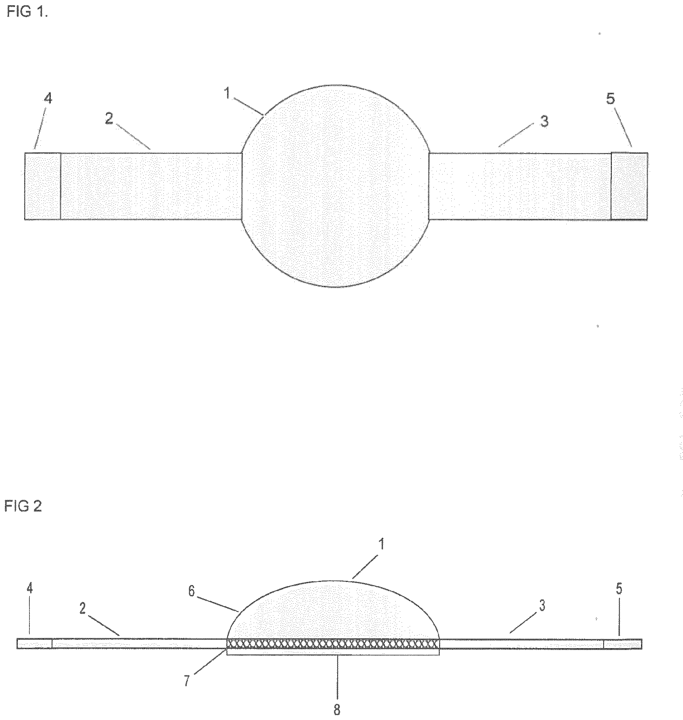

In view of figures 1 and 2, it can be observed that the protective device for the cellists' thig that dampens the harm of the cellos C bouts (12,13,14,15,16 and 17) is formed by a central cushion piece (1). This central piece contains a semi-rigid disc (7). The thig protector for the cellists' legs is held in place by two elastic strips (2 and 3) that allow to adjust the size and which is provided with a closing and opening system made of Velcro (4 and 5).

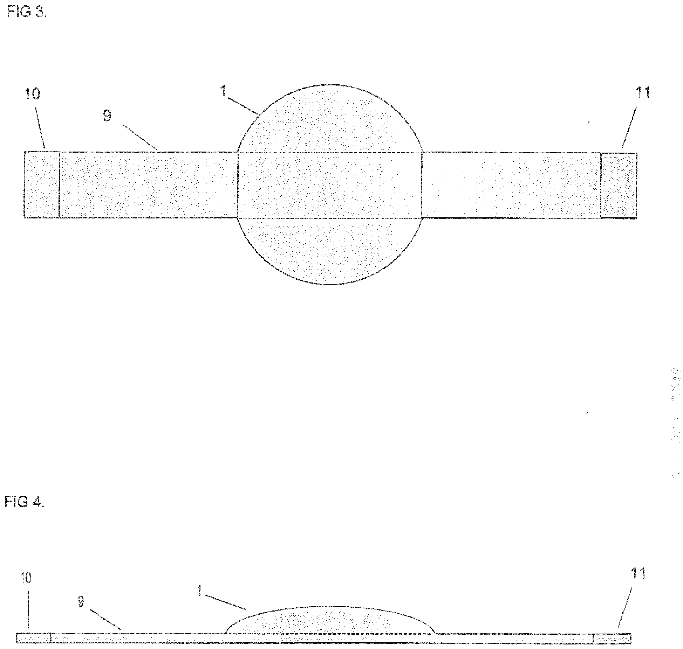

In view of the above figures 3 and 4, it can be observed how the acoustic cushion for the Cellist, Viola da gamba and Double Bass players' legs consists of a circular cushion structure (1). This structure is manufactured with a plastic substance whose acoustic properties inhibit the vibration transmission from the instrument to the musicians' body avoiding the loss of timbre richness, power, projection and sound quality. The acoustic cushion is adjusted by an elastic band (9) which can be adjusted to the musicians' leg and whose ends have a closing and opening system made with Velcro (10 and 11).

## Claims

1. Device for the Cellist, Viola da gamba and Double Bass players' legs **characterized by** the following pieces: a cushion body (1), and a leg fastening system with a closing and opening system.

2. Device **characterized by** the cushion body (1) which is formed by three parts which are: a semi-elliptical body filled with gel in its upper part (6) a semi-rigid disc in the intermediate part (7) and a base filled with gel in its lower part (8), and because the fastening means are two elastic strips (2 and 3) sewed or fixed to the central piece at the semi-rigid disc level (7).

3. Device **characterized by** the strips (2 and 3) that will have a fixed closing and opening system at the ends, sewed and stuck, which consists of the piece (4) male Velcro and the piece (5) female Velcro.

4. The device of claim 2 will be manufactured in different sizes according to the age and physical constitution of the cellist.

5. The device of claim 1 is **characterized by** the cushion body (1) composed by a plastic substance with acoustic properties, with an inner belt that can be attached to the leg and which allows the instrument to rest naturally thanks to its upper part (12, 13, 14, 15, 16 and 17) and the fastening system, which consists of an elastic band (9) with a closing and opening system (10 and 11).

6. The device of claim 5 is characterized because the cushion body (1) is composed of polyurethane foam.

7. The device of claim 5 is **characterized by** an adjustment that consists of a fabric band (9) tied to the central piece.

8. The device of claim 5 is characterized because the band (9) will have a closing and opening system fixed to it at the ends (10 and 11) which shall preferably consist of velcros.

9. The device of claims 5 to 8 will be manufactured in different sizes according to the morphological characteristics of the instrument, and to the age and physical constitution of the Cello, Viola da gamba or Double Bass player.
